(19) Europäisches Patentamt / European Patent Office / Office européen des brevets

(11) **EP 4 699 539 A1**

(12) **EUROPEAN PATENT APPLICATION**
published in accordance with Art. 153(4) EPC

(43) Date of publication:
**25.02.2026 Bulletin 2026/09**

(21) Application number: **23934115.9**

(22) Date of filing: **21.04.2023**

(51) International Patent Classification (IPC):
***A61B 6/00*** (2024.01)

(52) Cooperative Patent Classification (CPC):
**A61B 6/00**

(86) International application number:
**PCT/JP2023/015979**

(87) International publication number:
**WO 2024/218973 (24.10.2024 Gazette 2024/43)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **NEC Corporation
108-8001 Tokyo (JP)**

(72) Inventor: **SHINO, Ryosaku
Tokyo 108-8001 (JP)**

(74) Representative: **Betten & Resch
Patent- und Rechtsanwälte PartGmbB
Maximiliansplatz 14
80333 München (DE)**

(54) **IMAGE PROCESSING DEVICE, IMAGE PROCESSING METHOD, AND STORAGE MEDIUM**

(57) An image processing device includes a detection means 31X, an acquisition means 32X, a deformation means 33X, and a display control means 34X. The detection means 31X is configured to detect a dense portion where tissue is dense from a chest image. The acquisition means 32X is configured to acquire a first image obtained by cutting out an area based on the dense portion from the chest image. The deformation means 33X is configured to deform the first image based on information related to density of the tissue in the first image. The display control means 34X is configured to display, on a display device, a second image obtained by deforming the first image. It can be used to assist a user in decision making and the like.

Fig. 11

EP 4 699 539 A1

**Description**

TECHNICAL FIELD

The present disclosure relates to a technical field of an image processing device, an image processing method, and a storage medium that perform image processing.

BACKGROUND

Conventionally, an image processing system that performs processing on a chest image such as a mammography image by computer image analysis is known. For example, PTL 1 discloses an information processing system that generates and displays overlooking suppression data including image data indicating an area where a lesion is likely to be overlooked based on a mammography image and additional data.

CITATION LIST

PATENT LITERATURE

PTL 1: JP 2021-170174 A

SUMMARY

PROBLEM TO BE SOLVED

Since the area where the lesion is likely to be overlooked is usually a portion where tissue is dense and visibility is low, even when the area where the lesion is likely to be overlooked is detected and displayed as in PTL 1, it is difficult for an inspector to confirm whether there is an abnormality in the displayed area.

In view of the above-described issue, an object of the present disclosure is to provide an image processing device, an image processing method, and a storage medium capable of suitably displaying a portion where tissue is dense in a chest image.

MEANS FOR SOLVING THE PROBLEM

One mode of the image processing device is an image processing device including:

a detection means for detecting a dense portion where tissue is dense from a chest image;
an acquisition means for acquiring a first image obtained by cutting out an area based on the dense portion from the chest image;
a deformation means for deforming the first image based on information related to density of the tissue in the first image; and
a display control means for displaying, on a display device, a second image obtained by deforming the first image.

One mode of the image processing method is an image processing method executed by a computer, includes:

detecting a dense portion where tissue is dense from a chest image;
acquiring a first image obtained by cutting out an area based on the dense portion from the chest image;
deforming the first image based on information related to density of the tissue in the first image; and
displaying, on a display device, a second image obtained by deforming the first image.

One mode of the storage medium is a storage medium storing a program executed by a computer, the program causing the computer to execute processing including:

detecting a dense portion where tissue is dense from a chest image;
acquiring a first image obtained by cutting out an area based on the dense portion from the chest image;
deforming the first image based on information related to density of the tissue in the first image; and
displaying, on a display device, a second image obtained by deforming the first image.

EFFECT

An example advantage according to the present invention is to suitably display a portion where tissue is dense in a chest image.

BRIEF DESCRIPTION OF THE DRAWINGS

[Fig. 1] Fig. 1 illustrates a schematic configuration of a chest image processing system.
[Fig. 2] Fig. 2 is a diagram illustrating an outline of image processing executed by an image processing device in a first example embodiment.
[Fig. 3] Fig. 3 is an example of functional blocks of a processor of the image processing device in the first example embodiment.
[Figs. 4A and 4B] Fig. 4A illustrates an example of a target tissue mask image. Fig. 4B illustrates a target tissue density image calculated based on the target tissue mask image.
[Figs. 5A and 5B] Fig. 5A illustrates the target tissue density image. Fig. 5B is a diagram visualizing a deformation vector for each pixel calculated based on the target tissue density image.
[Figs. 6A and 6B] Fig. 6A illustrates the target tissue density image clearly indicating target tissue density for each pixel. Fig. 6B illustrates a correspondence relationship between each pixel of the target tissue density image and X-Y coordinates in a case where an area of the target tissue density image is regarded as an X-Y coordinate space.

[Fig. 7] Fig. 7 illustrates a first display example of a display screen.

[Fig. 8] Fig. 8 illustrates a second display example of the display screen.

[Fig. 9] Fig. 9 illustrates a third display example of the display screen.

[Fig. 10] Fig. 10 is an example of a flowchart indicating an outline of processing executed by the image processing device in the first example embodiment.

[Fig. 11] Fig. 11 is a block diagram of an image processing device in a second example embodiment.

[Fig. 12] Fig. 12 is an example of a flowchart executed by the image processing device in the second example embodiment.

EXAMPLE EMBODIMENT

Hereinafter, example embodiments of an image processing device, an image processing method, and a storage medium will be described with reference to the drawings.

<First Example Embodiment>

(1) System Configuration

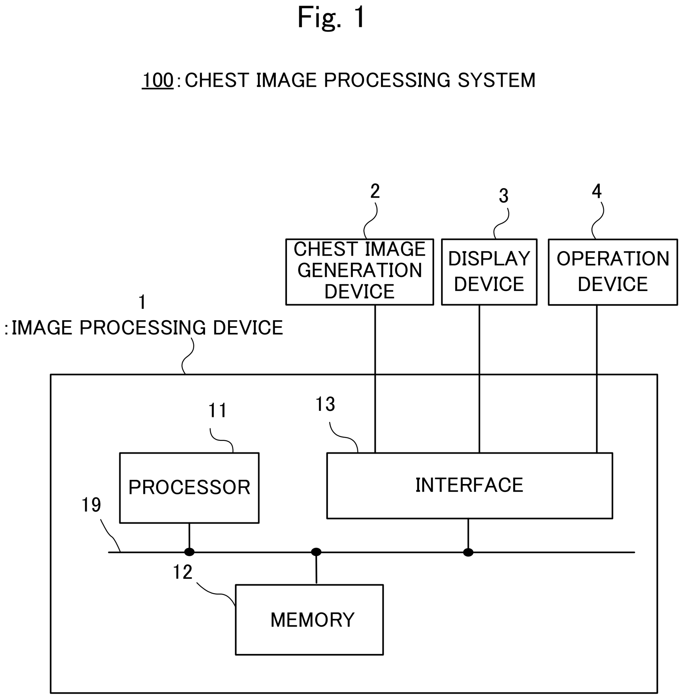

Fig. 1 illustrates a schematic configuration of a chest image processing system 100. As illustrated in Fig. 1, the chest image processing system 100 is a system that deforms an image representing a portion where density of tissue is high and visibility is low in a chest image of a patient as a subject by non-rigid deformation in such a way as to increase the visibility, and presents the deformed image to an inspector. The chest image processing system 100 mainly includes an image processing device 1, a chest image generation device 2, a display device 3, and an operation device 4.

The image processing device 1 performs, based on a chest image of a patient supplied from the chest image generation device 2, detection of a dense portion of tissue, cutting out an area based on the detected dense portion, deformation of a cut out image, and the like. The image processing device 1 also performs display control to cause the display device 3 to display information based on the deformed image, and performs various types of processing based on an operation signal received from the operation device 4.

The chest image generation device 2 generates a chest image obtained by imaging a chest that is a part to be imaged of a patient, and supplies the generated chest image to the image processing device 1. For example, the chest image generation device 2 generates the chest image (an X-ray image of a breast of the patient) by performing X-ray imaging for irradiating the breast with X-rays and detecting the X-rays transmitted through the breast. The chest image generation device 2 may be a mammography device based on a computed radiography (CR) method or a mammography device based on a flat panel detector (FPD) method. The chest image generated by the chest image generation device 2 is digital data that can be analyzed by the image processing device 1. For example, a pixel of the chest image is displayed as a larger pixel value (luminance) as X-ray absorption is larger, and is drawn white as the pixel value is larger on the chest image. The chest image generated by the chest image generation device 2 is not limited to the mammography image, and may be an ultrasound image, an MRI image, a CT image, a chest X-ray image, an angiography image, or the like.

In addition to the chest image, the chest image generation device 2 may supply, to the image processing device 1, at least one of patient information or inspection information of the patient as a subject of the chest image. In this case, examples of the patient information include patient identification information (patient ID) for identifying the patient and other attribute information (name, gender, date of birth, and the like) of the patient. Examples of the inspection information include inspection identification information (inspection ID) for identifying inspection, inspection date and time information, and information related to an inspection condition (inspection part, laterality (left, right), direction (for example, craniocaudal (CC), medio-lateral oblique (MLO), and a compressed breast thickness)).

The display device 3 performs predetermined display based on a display signal supplied from the image processing device 1. Examples of the display device 3 include a display such as a cathode ray tube (CRT) or a liquid crystal display (LDC) and a projector.

The operation device 4 generates an operation signal based on an operation by a user of the image processing device 1, such as a doctor. Examples of the operation device 4 include a button, a keyboard, a pointing device such as a mouse, a touch panel, a remote controller, an audio input device, and other optional user interface.

Fig. 1 also illustrates an example of a hardware configuration of the image processing device 1. The image processing device 1 mainly includes a processor 11, a memory 12, and an interface 13. These elements are connected via a data bus 19.

The processor 11 executes predetermined processing by executing a program or the like stored in the memory 12. The processor 11 is a processor such as a central processing unit (CPU), a graphics processing unit (GPU), or a tensor processing unit (TPU). The processor 11 may include a plurality of processors. The processor 11 is an example of a computer.

The memory 12 includes various types of volatile memories used as a working memory, such as a random access memory (RAM) and a read only memory (ROM), and a non-volatile memory that stores information necessary for processing of the image processing device 1. The memory 12 may include an external storage device such as a hard disk connected to or built in the image processing device 1, or may include a storage medium such as a freely attachable and detachable flash memory. The memory 12 stores a program and other information necessary for the image processing device 1 to execute each processing in the present example embodiment.

The interface 13 performs an interface operation between the image processing device 1 and an external device. For example, the interface 13 is electrically connected to the chest image generation device 2, the display device 3, and the operation device 4. The interface 13 may be a communication interface such as a network adapter for performing wired or wireless communication with the external device, or may be a hardware interface conforming to a universal serial bus (USB), serial AT attachment (SATA), or the like. The interface 13 may perform the interface operation with the external device such as the chest image generation device 2, the display device 3, and the operation device 4 via a communication network such as the Internet.

The configuration of the chest image processing system 100 illustrated in Fig. 1 is an example, and various changes may be made to the configuration.

For example, the image processing device 1 may be integrally configured with at least one of the chest image generation device 2, the display device 3, or the operation device 4. In another example, the image processing device 1 may include an audio output device that outputs information by audio. In still another example, the image processing device 1 may include a plurality of devices.

In another example, the chest image generation device 2 may be a storage device that stores a chest image of a patient generated in advance. In this case, the above storage device can perform data communication with the image processing device 1, and the image processing device 1 acquires the chest image of the patient specified by the operation device 4 from the storage device, for example, and executes processing based on the present example embodiment. The above-described storage device may be a server device that performs the data communication with the image processing device 1 via the communication network, or may be built in the image processing device 1 as the memory 12 of the image processing device 1.

(2) Description of Outline

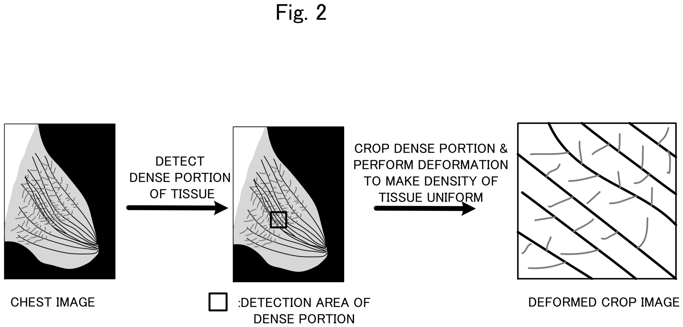

Fig. 2 is a diagram illustrating an outline of image processing executed by the image processing device 1 in the first example embodiment.

In a case where a chest image of a patient is acquired from the chest image generation device 2, the image processing device 1 detects a dense portion of tissue (also referred to as "target tissue") in a breast by performing image analysis on the acquired chest image. Here, the "target tissue" is tissue in the breast appearing in the chest image, and examples of the target tissue include a mammary gland and a mammary duct (including a mammary duct formed as a part of the mammary gland). In Fig. 2, a detection area of the dense portion is highlighted by a rectangular frame.

Next, the image processing device 1 acquires an image (also referred to as a "first crop image") obtained by cutting out an area in the chest image based on the detection area of the dense portion. The first crop image may be an image obtained by cutting out the detection area of the dense portion from the chest image, or may be an image obtained by cutting out an area smaller or larger than the detection area of the dense portion from the chest image in such a way as to be within a predetermined size.

The image processing device 1 then non-rigidly deforms the first crop image in such a way as to make density of the target tissue appearing in the first crop image uniform. Hereinafter, the image obtained by non-rigidly deforming the first crop image is also referred to as a "second crop image". In this case, the image processing device 1 deforms the first crop image in such a way as to make a degree of density of the target tissue on the image uniform. As a result, the second crop image becomes the image obtained by non-rigidly deforming the first crop image in such a way that the high density of the target tissue is eliminated. The image processing device 1 then causes the display device 3 to display information based on the second crop image. As a result, an inspector can suitably confirm the dense portion of the target tissue, which is difficult to confirm with the first crop image, with the second crop image.

(3) Functional Block

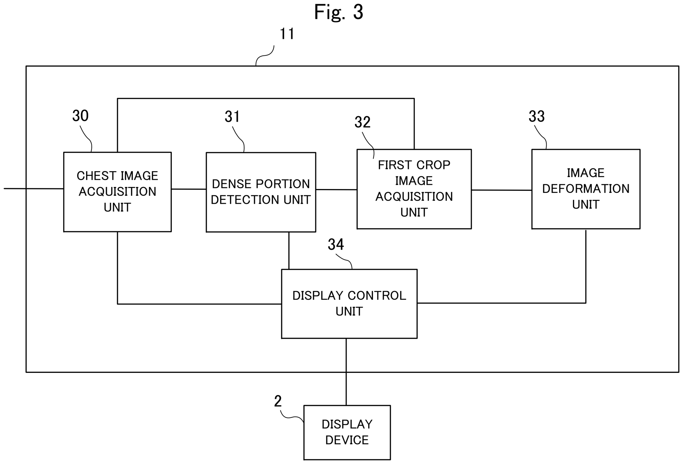

Fig. 3 is an example of functional blocks of the processor 11 of the image processing device 1 in the first example embodiment. The processor 11 of the image processing device 1 functionally includes a chest image acquisition unit 30, a dense portion detection unit 31, a first crop image acquisition unit 32, an image deformation unit 33, and a display control unit 34. While blocks that exchange data with each other are connected by a solid line in Fig. 3, a combination of the blocks that exchange data with each other is not limited to this. The same applies to diagrams of other functional blocks described later.

The chest image acquisition unit 30 receives a chest image of a patient generated by the chest image generation device 2 from the chest image generation device 2 via the interface 13. The chest image acquisition unit 30 then supplies the acquired chest image to the dense portion detection unit 31, the first crop image acquisition unit 32, and the display control unit 34.

The dense portion detection unit 31 detects a dense portion of target tissue from the chest image supplied from the chest image acquisition unit 30, and supplies a detection result to the first crop image acquisition unit 32.

Here, specific examples (a first example and a second example) of the detection of the dense portion will be sequentially described.

In the first example of the detection of the dense portion, the dense portion detection unit 31 infers the dense portion of the target tissue in the chest image based on an inference model (inference engine) that outputs a detection result of a dense portion of target tissue from an input chest image. The above-described inference model is, for example, a model that has been trained by machine learning, and is a model that has learned a relationship between a chest image input to the inference model and a dense portion of target tissue in the image. Specifically, the inference model is a model trained in such a way as to output, in a case where a chest image is input, an inference result of a dense portion of target tissue in the input chest image. The inference result output by the inference model may be position information (for example, coordinate information of a vertex in the case of a bounding box) indicating an area on the image surrounding the dense portion of the target tissue, or may be position information indicating a representative position on the image of the dense portion of the target tissue.

The inference model is trained by, for example, a training data set including a plurality of records that is a combination of a chest image as a sample and correct answer information indicating a dense portion that is a correct answer in the chest image. The inference model may be a model (including a statistical model, the same applies hereinafter) adopted in optional machine learning, such as a neural network or a support vector machine. Examples of a representative model of such a neural network include Fully Convolutional Network, SegNet, U-Net, V-Net, Feature Pyramid Network, Mask R-CNN, and DeepLab. In a case where the inference model is configured by the neural network, for example, various parameters such as a layer structure, a neuron structure of each layer, the number of filters and a filter size in each layer, and a weight of each element of each filter are stored in the memory 12 or the like as learned parameters.

The above-described inference model may be trained in such a way as to detect, as the above-described dense portion, a portion where the target tissue is dense and that is suspected to be a lesion. In this case, the inference model is trained by a training data set including a plurality of records that is a combination of a chest image as a sample and correct answer information indicating a portion where the target tissue is dense and that is suspected to be a lesion in the chest image.

The inference model may output inference results indicating a plurality of dense portions of the target tissue. In a case where the inference model is configured by the neural network, the inference model may output, as the inference results, certainty factors for the detected dense portions together with position information of the detected dense portions. In this case, for example, the dense portion detection unit 31 adopts the inference result of the dense portion where the certainty factor is equal to or more than a predetermined threshold as the detection result of the dense portion to be used in the subsequent processing, and supplies the detection result to the first crop image acquisition unit 32 and the display control unit 34.

In the second example of the detection of the dense portion, the dense portion detection unit 31 calculates a degree of density of the target tissue (also referred to as "target tissue density") for each pixel of the chest image, and detects the dense portion based on the target tissue density. In this case, for example, the dense portion detection unit 31 may detect, as the dense portion, an area where equal to or more than a predetermined number of pixels whose target tissue density is equal to or more than a predetermined threshold are connected. The above-described predetermined threshold and predetermined number are stored in, for example, the memory 12 or the like. A method of detecting the target tissue and a method of calculating the target tissue density are the same as a method of detecting the target tissue and a method of calculating the target tissue density executed by the image deformation unit 33, and details will be described later.

The first crop image acquisition unit 32 performs processing of cutting out a first crop image from a chest image supplied from the chest image acquisition unit 30 based on a detection result of a dense portion of target tissue supplied from the dense portion detection unit 31, and supplies the cut out first crop image to the image deformation unit 33.

In this case, for example, in a case where the detection result of the dense portion of the target tissue supplied from the dense portion detection unit 31 indicates an area in the chest image, the first crop image acquisition unit 32 acquires the first crop image obtained by cutting out the area indicated by the detection result of the dense portion. In this case, for example, the first crop image acquisition unit 32 may acquire the first crop image obtained by cutting out an area larger or smaller than the area indicated by the detection result of the dense portion from the chest image in such a way that the first crop image has a predetermined size determined in advance. In this case, for example, the first crop image acquisition unit 32 cuts out the first crop image from the chest image in such a way that a center of the area indicated by the detection result of the dense portion overlaps a center of the first crop image. In a case where the detection result of the dense portion of the target tissue supplied from the dense portion detection unit 31 indicates a representative position in the chest image, the first crop image acquisition unit 32 acquires, for example, the first crop image obtained by cutting out, from the chest image, an area having a predetermined size and a predetermined shape with the representative position as the center of the first crop image.

The image deformation unit 33 non-rigidly deforms a first crop image supplied from the first crop image acquisition unit 32 in such a way that density of target tissue for each pixel is made uniform, and supplies a second crop image obtained by deforming the first crop image to the display control unit 34. In this case, the image deformation unit 33 sequentially executes

> (a) detection of the target tissue in the first crop image,
> (b) calculation of target tissue density for each pixel in the first crop image,
> (c) calculation of a deformation vector field based on the target tissue density, and
> (d) deformation of the first crop image based on the deformation vector field (so-called image warping). Details of processing of the image deformation unit 33 will be described later.

The display control unit 34 controls display by the display device 3 based on a second crop image and the like supplied from the image deformation unit 33. In this case, by generating a display signal and supplying the generated display signal to the display device 3, the display control unit 34 causes the display device 3 to display the second crop image and the like. The display control unit 34 may display, on the display device 3 together with the second crop image, a chest image clearly indicating a detection area of a dense portion detected by the dense portion detection unit 31 based on information supplied from the chest image acquisition unit 30 and the dense portion detection unit 31. A display example displayed by the display device 3 based on the control by the display control unit 34 will be described later.

The components of the chest image acquisition unit 30, the dense portion detection unit 31, the first crop image acquisition unit 32, the image deformation unit 33, and the display control unit 34 can be achieved by, for example, the processor 11 executing a program. Each component may also be achieved by recording a necessary program in an optional non-volatile storage medium and installing the program as necessary. At least a part of these components is not limited to be achieved by software by the program, and may be achieved by a combination of any of hardware, firmware, and software, or the like. At least a part of these components may also be achieved by using, for example, a user-programmable integrated circuit such as a field-programmable gate array (FPGA) or a microcontroller. In this case, a program including the above components may be achieved by using the integrated circuit. At least a part of the components may include an application specific standard produce (ASSP), an application specific integrated circuit (ASIC), or a quantum processor (quantum computer control chip). In this manner, the components may be achieved by various types of hardware. The same applies to other example embodiments described later. These components may also be achieved by, for example, cooperation of a plurality of computers by using a cloud computing technology or the like.

(4) Details of Image Deformation

Next, each of the above-described processing of (a) to (d) included in the image deformation processing by the image deformation unit 33 will be described.

First, "(a) detection of the target tissue in the first crop image" will be described.

The image deformation unit 33 generates a mask image indicating presence or absence of the target tissue for each pixel by executing, for each pixel of the first crop image, threshold processing of a luminance value (pixel value) associated with each pixel. In this case, the image deformation unit 33 generates the mask image in which a pixel having a luminance value higher than a predetermined threshold and a pixel having a luminance value equal to or less than the predetermined threshold have different pixel values. The above-described threshold is stored in advance in the memory 12 or the like, for example.

Preferably, the image deformation unit 33 performs optional filter processing (edge detection processing) in addition to the above-described threshold processing of the luminance value. A filter used for the above-described filter processing may be, for example, an optional filter such as Canny, Sobel, or Laplacian. In this case, for example, the image deformation unit 33 acquires an image obtained by inputting an image obtained by the threshold processing of the luminance value to the above-described filter as the mask image indicating the presence or absence of the target tissue for each pixel.

Hereinafter, the mask image obtained by the processing of (a) is referred to as an "target tissue mask image", a pixel corresponding to the target tissue in the target tissue mask image is referred to as an "target tissue pixel", and a pixel not corresponding to the target tissue is also referred to as a "non-target tissue pixel". The target tissue mask image is an example of "information indicating presence or absence of tissue for each unit area".

Next, "(b) calculation of target tissue density for each pixel in the first crop image" will be described.

The image deformation unit 33 calculates the target tissue density for each pixel of the first crop image by using the target tissue mask image. In this case, the image deformation unit 33 selects one pixel at a time from the first crop image, detects a target tissue pixel within an offset distance for each selected pixel, and determines a total value of weights according to a distance between the detected target tissue pixel and the selected pixel as the target tissue density of the selected pixel. Hereinafter, an image in which the target tissue density calculated by the processing of (b) is set as a pixel value of each pixel is also referred to as an "target tissue density image".

Granularity of the target tissue mask image and the target tissue density image does not need to be granularity in units of one pixel in a chest image, and may be granularity in units of a plurality of pixels or in units of subpixels. Instead of calculating the target tissue density that becomes a higher value as the degree of density of the target tissue is higher, the image deformation unit 33 may calculate an index that becomes a lower value as the degree of density of the target tissue is higher, and may use the index instead of the target tissue density. The same applies to a case where the dense portion detection unit 31 calculates the target tissue density in the detection of the dense portion. The target tissue density image is information indicating a distribution of the target tissue density for each unit area (one pixel, a plurality of pixels, or a subpixel), and is an example of "distribution information".

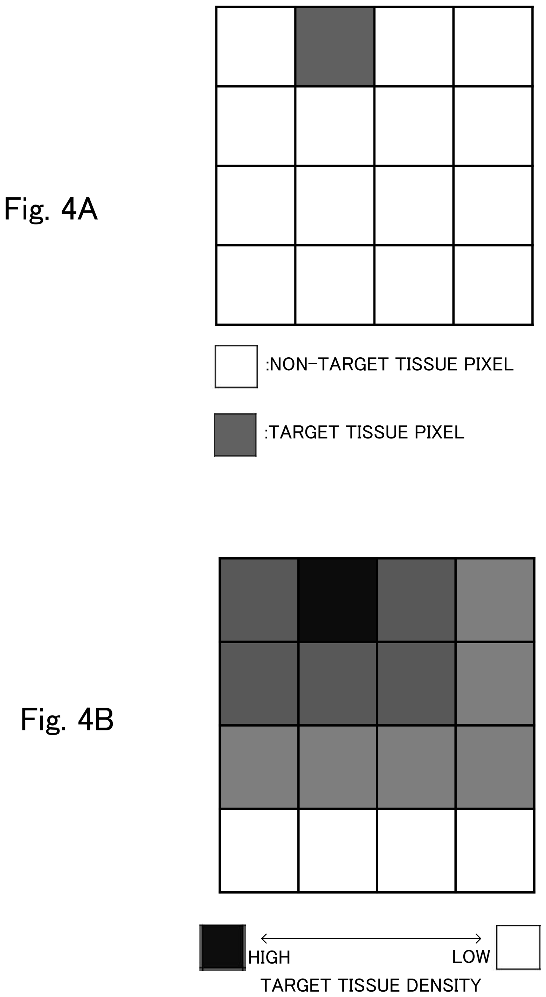

Fig. 4A illustrates an example of the target tissue mask image, and Fig. 4B illustrates the target tissue density image calculated based on the target tissue mask image of Fig. 4A. Here, in order to simplify the description, the target tissue mask image is assumed to be a $4 \times 4$ image, a pixel in the first row and the second column is the target tissue pixel, and the other pixels are the non-target tissue pixels.

In this case, the image deformation unit 33 selects all the pixels of the target tissue mask image one by one. Then, in a case where there is the target tissue pixel within a predetermined offset distance (here, a distance of two pixels) from the selected pixel, the image deformation unit 33 adds a value according to a distance between the selected pixel and the target tissue pixel as the target tissue density of the selected pixel. The "value according to the distance" increases as the distance decreases, and becomes maximum in a case where the distance is 0 (that is, the selected pixel and the target tissue pixel are the same pixel). In the examples of Figs. 4A and 4B, the target tissue density of the pixel in the first row and the second column, which is the target tissue pixel, becomes a first value that is a maximum value, the target tissue density of a pixel separated by one pixel from the pixel in the first row and the second column becomes a second value that is the second highest, the target tissue density of a pixel separated by two pixels from the pixel in the first row and the second column becomes a third value that is lower than the second value, and the target tissue density of the other pixels becomes 0.

In a case where there is a plurality of target tissue pixels within the offset distance, the image deformation unit 33 sets a total value of values according to distances between the selected pixel and the plurality of target tissue pixels as the target tissue density of the selected pixel.

As described above, the image deformation unit 33 can suitably calculate the target tissue density. The granularity for calculating the target tissue density (that is, the granularity of the target tissue density image) does not need to be one pixel, and may be a plurality of pixels or a subpixel.

Next, "(c) calculation of a deformation vector field based on the target tissue density" will be described.

The image deformation unit 33 solves a diffusion equation by regarding an area of the target tissue density image as a solvent and assuming that a solute having the target tissue density indicated by the target tissue density image as concentration is dissolved in the solvent, and calculates a movement vector of each pixel to the next step (that is, a dissolved state). This movement vector relates to a deformation vector, and a space in which the deformation vectors are arranged in the area of the target tissue density image relates to a deformation vector field. The deformation vector is a vector for matching an optional point on the first crop image that is an image to be deformed with a related point on the second crop image that is a target image.

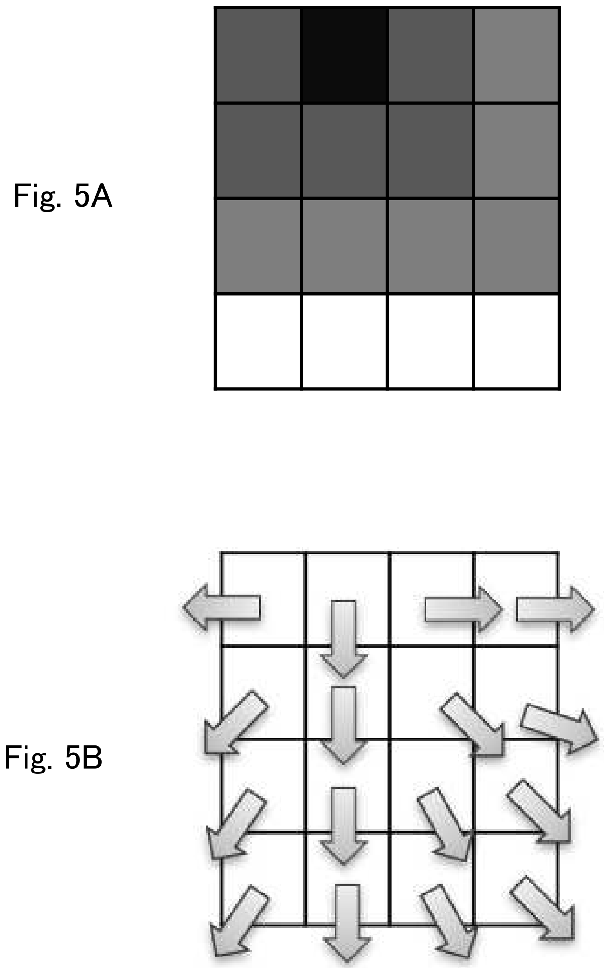

Fig. 5A illustrates the target tissue density image, and Fig. 5B is a diagram visualizing the deformation vector for each pixel calculated based on the target tissue density image illustrated in Fig. 5A. As illustrated in Figs. 5A and 5B, each deformation vector indicates a direction along a direction from a position where the target tissue density is high (that is, the concentration is high) to a position where the target tissue density is low. In this manner, the deformation vector that diffuses the dense portion of the target tissue is calculated in such a way that the density of the target tissue is made uniform.

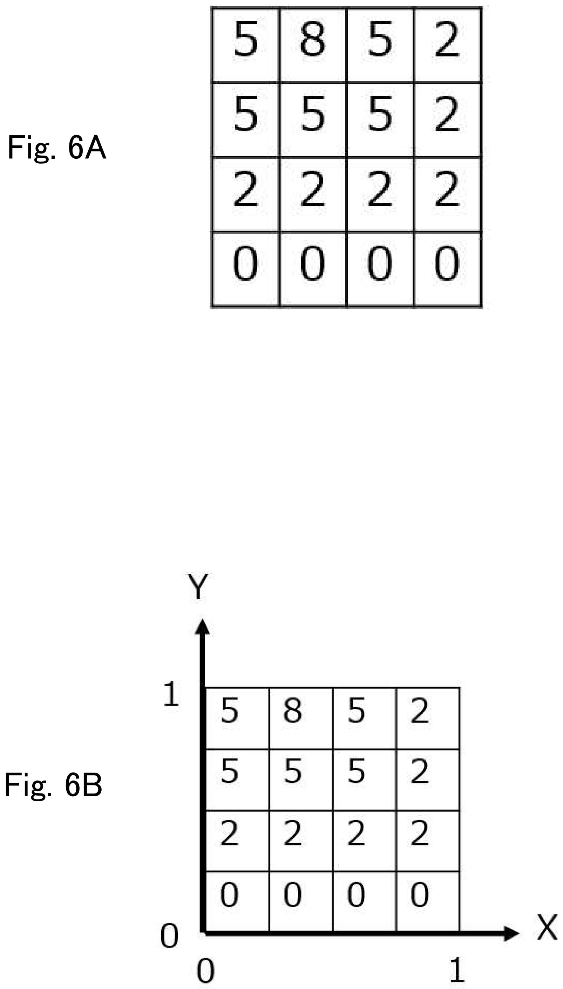

Here, a specific example of a method of calculating the deformation vector field will be described with reference to Figs. 6A and 6B. Fig. 6A illustrates the target tissue density image in which the target tissue density for each pixel is indicated by a numerical value, and Fig. 6B illustrates a correspondence relationship between the target tissue density image and X-Y coordinates in a case where an area of the target tissue density image is regarded as an X-Y coordinate space. Then, by substituting each value of "u(x, y, t)" specified by Fig. 6B into a diffusion equation of the target tissue density "u(x, y, t)" indicated by the following expression, the image deformation unit 33 solves the diffusion equation.

[Expression 1]

$$\frac{\partial u}{\partial t} = C \left( \frac{\partial^2 u}{\partial x^2} + \frac{\partial^2 u}{\partial y^2} \right)$$

Coordinate values having a value range of 0 to 1 in the X-Y coordinate space are indicated by "x" and "y", a time is indicated by "t", and an initial state related to the target tissue density image is indicated by "t = 0". The movement vector (that is, the deformation vector) on the area of the target tissue density image is indicated by "C". The image deformation unit 33 then fixes a value of one point (for example, the origin) among ends of the area of the target tissue density image. For example, the image deformation unit 33 sets "u(0, 0, t) = 0". Then, in the case of solving the diffusion equation, the image deformation unit 33 uses the following values as inputs according to Fig. 6B.

$$u(0, 1, 0) = 5,$$

$$u(1/3, 1, 0) = 8,$$

$$u(2/3, 1, 0) = 5,$$

$$u(1, 1, 0) = 2,$$

$$u(0, 2/3, 0) = 5,$$

$$u(1/3, 2/3, 0) = 5,$$

$$u(2/3, 2/3, 0) = 5,$$

$$u(1, 2/3, 0) = 2,$$

$$u(0, 1/3, 0) = 2,$$

$$u(1/3, 1/3, 0) = 2,$$

$$u(2/3, 1/3, 0) = 2,$$

$$u(1, 1/3, 0) = 2,$$

$$u(0, 0, 0) = 0,$$

$$u(1/3, 0, 0) = 0,$$

$$u(2/3, 0, 0) = 0,$$

and

$$u(1, 0, 0) = 0.$$

The image deformation unit 33 then solves the above-described diffusion equation by approximating a differential to a difference form by a difference method. As a result, the image deformation unit 33 can suitably calculate the movement vector (that is, the deformation vector) in the target tissue density image.

Next, "(d) deformation of the first crop image based on the deformation vector field" will be described.

The image deformation unit 33 regards the movement vector for each pixel on the target tissue density image calculated by the processing of (c) as the deformation vector field from the first crop image to the second crop image, and deforms the first crop image based on the deformation vector field. In this case, the image deformation unit 33 may use, for example, an optional image warping technology such as Backward Image Warping. As a result, the image deformation unit 33 can acquire the second crop image obtained by deforming the first crop image in such a way as to make the target tissue density uniform.

(5) Display Example

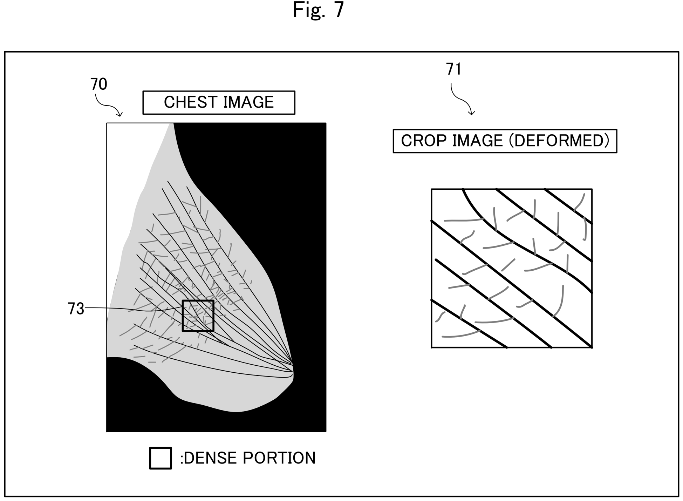

Fig. 7 illustrates a first display example of a display screen displayed on the display device 3 by the display control unit 34. The display control unit 34 outputs, to the display device 3, display information generated based on the chest image supplied from the chest image acquisition unit 30, the second crop image supplied from the image deformation unit 33, and the like. The display control unit 34 causes the display device 3 to display the display screen illustrated in Fig. 7 by transmitting the display information to the display device 3.

In the first display example illustrated in Fig. 7, the display control unit 34 provides a chest image display area 70 and a second crop image display area 71 on the display screen.

Here, the display control unit 34 displays the chest image acquired by the chest image acquisition unit 30 from the chest image generation device 2 in the chest image display area 70. On the chest image, the display control unit 34 superimposes and displays a frame 73 that emphasizes the detection area of the dense portion on the chest image based on a detection result of the dense portion supplied from the dense portion detection unit 31. The display control unit 34 may display, based on information supplied from first crop image acquisition unit 32, a frame that emphasizes a related area of a first crop image as the frame 73.

The display control unit 34 displays the second crop image supplied from the image deformation unit 33 in the second crop image display area 71. In this second crop image, a degree of density of object cells such as mammary ducts is reduced, and visibility of the object cells is enhanced. Therefore, an inspector as a viewer can confirm in detail, in the second crop image display area 71, presence or absence of an abnormality such as a lesion particularly at a portion where the object cells are dense in the chest image. The viewer can also grasp a portion related to the second crop image in an entire chest of a patient by referring to the chest image display area 70 in which the frame 73 is illustrated.

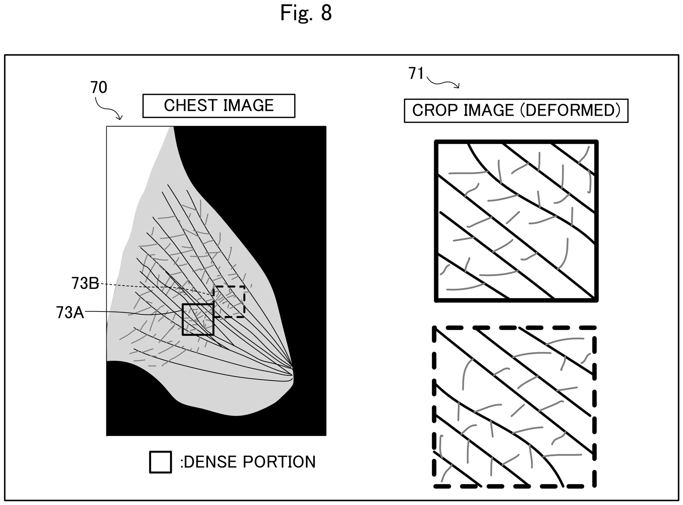

Fig. 8 illustrates a second display example of the display screen displayed on the display device 3 by the display control unit 34. In the second display example, the image processing device 1 detects a plurality of the dense portions of the object cells, and generates and displays the second crop images related to the detected dense portions. In the second display example, the display control unit 34 provides the chest image display area 70 and the second crop image display area 71 on the display screen.

In the second display example, since the dense portion detection unit 31 detects the plurality of dense portions, the display control unit 34 displays, on the chest image, a frame 73A and a frame 73B that emphasize the detection areas of the dense portions detected by the dense portion detection unit 31. In this case, the image deformation unit 33 acquires the second crop images obtained by deforming the first crop image related to the detection areas of the dense portions, and the display control unit 34 displays the second crop images supplied from the image deformation unit 33 in the second crop image display area 71 in association with the frames 73A and 73B indicating the detection areas of the related dense portions. In the second display example, as an example, the display control unit 34 emphasizes, with a solid line frame, an outer frame of the second crop image related to the detection area of the dense portion indicated by the frame 73A that is a solid line frame, and emphasizes, with a broken line frame, an outer frame of the second crop image related to the detection area of the dense portion indicated by the frame 73B that is a broken line frame.

In this manner, even in a case where there is the plurality of detection areas of the dense portions, the display control unit 34 can suitably present the second crop images obtained by the deformation in such a way as to improve the visibility of the object cells at the dense portions. Instead of the example of Fig. 8, the display control unit 34 may receive selection of the frame 73A or 73B by a user on the display screen, and display only the second crop image related to the selected frame in the second crop image display area 71. As a result, the display control unit 34 can clearly display the second crop image related to the portion of interest to the viewer in the second crop image display area 71.

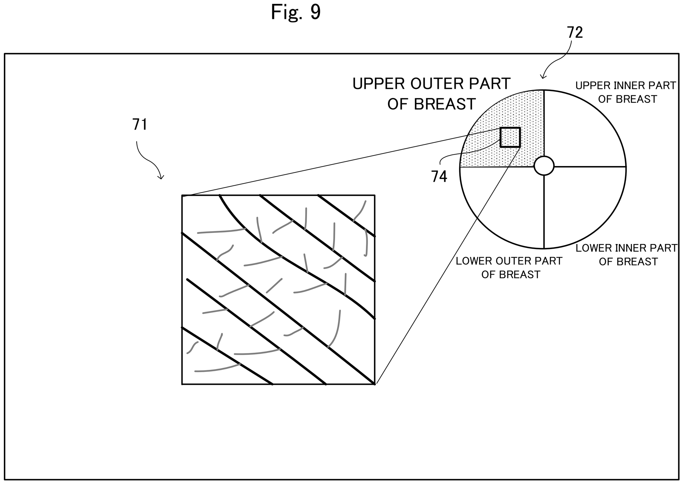

Fig. 9 illustrates a third display example of the display screen displayed on the display device 3 by the display control unit 34. In the third display example, instead of displaying the chest image displayed in the first display example and the second display example, the image processing device 1 displays information indicating a relative position of the second crop image to be displayed in the entire chest (specifically, breast). In the third display example, the display control unit 34 provides the second crop image display area 71 and a crop portion display area 72 on the display screen.

In this case, the display control unit 34 displays the second crop image supplied from the image deformation unit 33 in the second crop image display area 71, and displays a schematic view of the breast in which the breast is divided into four areas (an upper outer part, a lower outer part, an upper inner part, and a lower inner part) in the crop portion display area 72. In the crop portion display area 72, the display control unit 34 emphasizes the area of the breast "the upper outer part of the breast" corresponding to the portion indicated by the second crop image, and emphasizes the portion indicated by the second crop image with a frame 74. In this case, for example, the display control unit 34 may specify the relative position of the portion indicated by the second crop image in the entire breast by using an optional method of obtaining a three-dimensional position of a portion related to an optional image area specified from a chest image (including a method of three-dimensionally reconfiguring a chest from the chest image). In another example, in a case where information indicating a correspondence relationship between each pixel of the chest image and the schematic view of the breast is stored in advance in the memory 12 or the like, the display control unit 34 may refer to the information and specify the relative position of the portion indicated by the second crop image in the entire breast.

In this manner, in the third display example, even in a case where the chest image is not displayed, the display control unit 34 can cause the viewer to confirm presence or absence of an abnormality particularly in the portion where the object cells are dense in the chest image while causing the viewer to grasp the relative position of the portion related to the second crop image in the entire chest of the patient. In each display example, the display control unit 34 may further display information related to the patient or inspection on the display screen based on at least one of patient information or inspection information received together with the chest image from the chest image generation device 2. For example, a coping method may be determined based on a model generated by machine learning of a correspondence relationship between inspection information and a coping method for a chest image and the inspection information of the patient, and the coping method may be further displayed on the display screen. The method of determining the coping method is not limited to the above-described method.

(7) Processing Flow

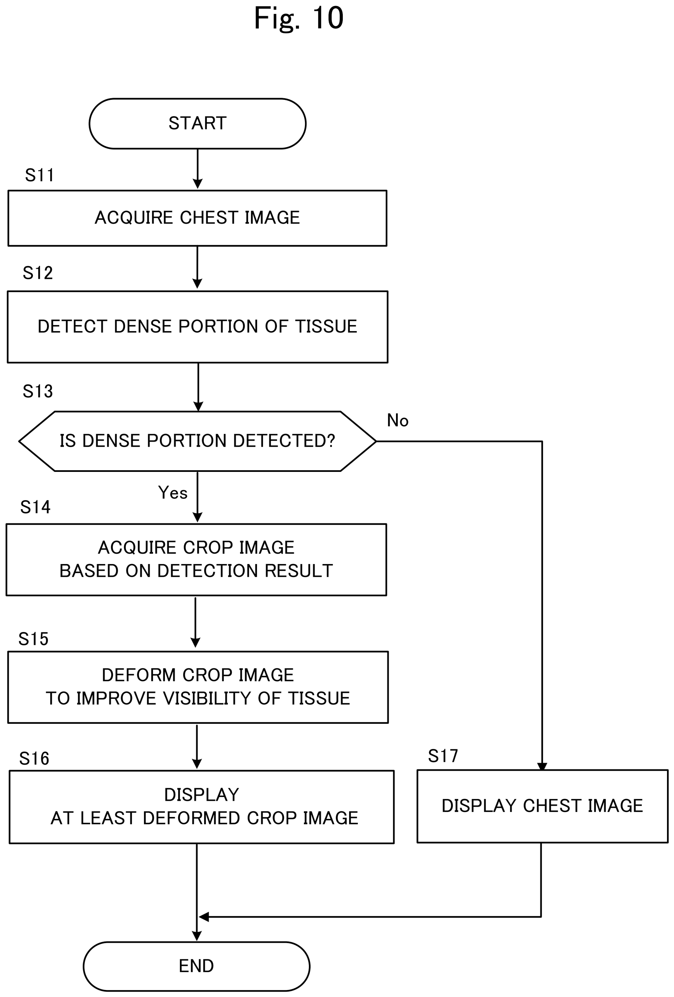

Fig. 10 is an example of a flowchart indicating an outline of processing executed by the image processing device 1 in the first example embodiment.

First, the image processing device 1 acquires a chest image obtained by imaging a chest of a patient from the chest image generation device 2 (step S11). Next, the image processing device 1 detects a dense portion of target tissue from the chest image acquired in step S11 (step S12).

The image processing device 1 then determines whether the dense portion of the target tissue is detected from the chest image (step S13). Then, in a case where it is determined that the dense portion of the target tissue is detected from the chest image (step S13; Yes), the image processing device 1 acquires a first crop image cut out from the chest image based on a detection result of the dense portion of the target tissue (step S14). The image processing device 1 then deforms the first crop image in such a way as to improve visibility of the target tissue (step S15). In this case, the image processing device 1 generates a target tissue density image that is a map of target tissue density in the first crop image, and acquires a second crop image obtained by deforming the first crop image in such a way that the target tissue density is made uniform based on the target tissue density image.

After step S15, the image processing device 1 displays the second crop image at least on the display device 3 (step S16). In this case, for example, the image processing device 1 causes the display device 3 to display the display screen based on any one of the first display example to the third display example. On the other hand, in a case where it is determined that the dense portion of the target tissue is not detected from the chest image in step S13 (step S13; No), the image processing device 1 causes the display device 3 to display the chest image (step S17). In this case, since there is no dense portion of the target tissue that is difficult for an inspector to visually recognize, the inspector can suitably confirm a state of the chest of the patient based on the chest image.

<Second Example Embodiment>

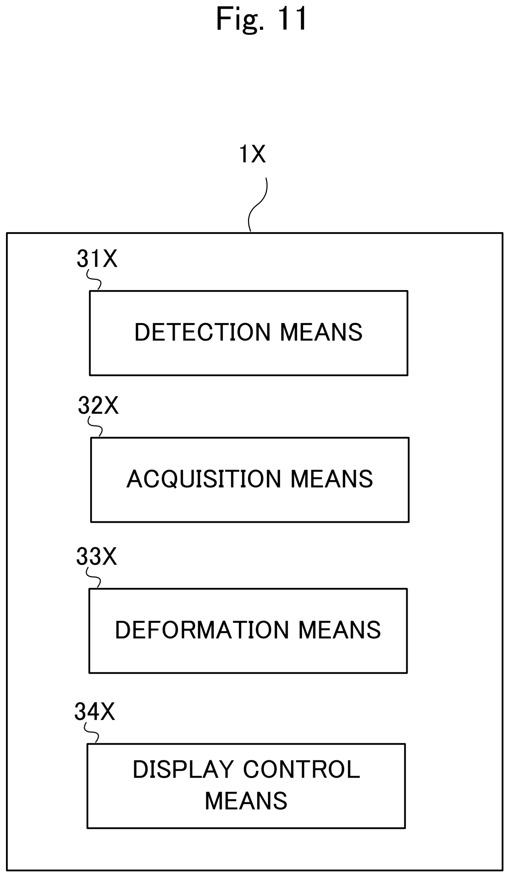

Fig. 11 is a block diagram of an image processing device 1X according to the second example embodiment. The image processing device 1X includes a detection means 31X, an acquisition means 32X, a deformation means 33X, and a display control means 34X. The image processing device 1X may be configured by plural devices.

The detection means 31X is configured to detect a dense portion where tissue is dense from a chest image. Examples of the detection means 31X include the dense portion detection unit 31 according to the first example embodiment.

The acquisition means 32X is configured to acquire a first image obtained by cutting out an area based on the dense portion from the chest image. Examples of the "area based on the dense portion" include: the area directly corresponding to the dense portion in the chest image; and the area zoomed in or out of the chest image based on the dense portion. Examples of the acquisition means 32X include the first crop image acquisition unit 32 according to the first example embodiment.

The deformation means 33X is configured to deform the first image based on information related to density of the tissue in the first image. Examples of the "information related to density of the tissue in the first image" include the "target tissue mask image" and the "target tissue density image" according to the first example embodiment.

The display control means 34X is configured to display, on a display device, a second image obtained by deforming the first image. The display device may be configured with the image processing device 1X as a single device or may be independent device separate from the image processing device 1X. Examples of the display control means 34X include the display control unit 34 according to the first example embodiment.

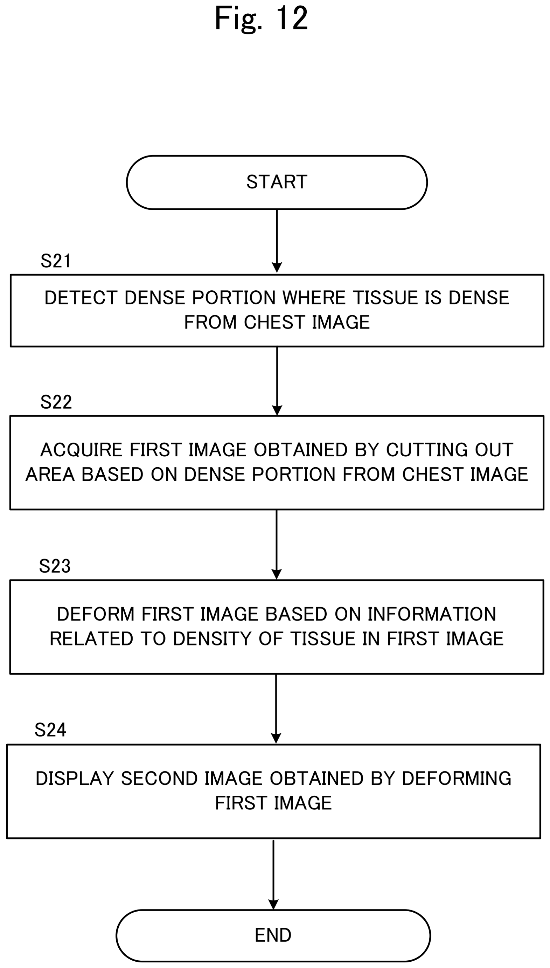

Fig. 12 illustrates an example of the flowchart indicative of a procedure of the process in the second example embodiment. The detection means 31X detects a dense portion where tissue is dense from a chest image (step S21). The acquisition means 32X acquires a first image obtained by cutting out an area based on the dense portion from the chest image (step S22). The deformation means 33X deforms the first image based on information related to density of the tissue in the first image (step S23). The display control means 34X displays, on a display device, a second image obtained by deforming the first image (step S24).

According to the second example embodiment, the image processing device 1X deforms the first image based on information related to density of the tissue to display the second image obtained by deforming the first image. This enables the user to suitably confirm the state of the dense portion where tissue is dense.

In the example embodiments described above, the program is stored by any type of a non-transitory computer-readable medium (non-transitory computer readable medium) and can be supplied to a control unit or the like that is a computer. The non-transitory computer-readable medium include any type of a tangible storage medium. Examples of the non-transitory computer readable medium include a magnetic storage medium (e.g., a flexible disk, a magnetic tape, a hard disk drive), a magnetic-optical storage medium (e.g., a magnetic optical disk), CD-ROM (Read Only Memory), CD-R, CD-R/W, a solid-state memory (e.g., a mask ROM, a PROM (Programmable ROM), an EPROM (Erasable PROM), a flash ROM, a RAM (Random Access Memory)). The program may also be provided to the computer by any type of a transitory computer readable medium. Examples of the transitory computer readable medium include an electrical signal, an optical signal, and an electromagnetic wave. The transitory computer readable medium can provide the program to the computer through a wired channel such as wires and optical fibers or a wireless channel.

The whole or a part of the example embodiments described above (including modifications, the same applies hereinafter) can be described as, but not limited to, the following Supplementary Notes.

[Supplementary Note 1]

An image processing device comprising:

a detection means for detecting a dense portion where tissue is dense from a chest image;
an acquisition means for acquiring a first image obtained by cutting out an area based on the dense portion from the chest image;
a deformation means for deforming the first image based on information related to density of the tissue in the first image; and
a display control means for displaying, on a display device, a second image obtained by deforming the first image.

[Supplementary Note 2]

The image processing device according to Supplementary Note 1, wherein the display control means displays, on the display device, the second image and information related to the area based on the dense portion.

[Supplementary Note 3]

The image processing device according to Supplementary Note 2, wherein the display control means displays, on the display device, the second image and the chest image in which the area based on the dense portion is emphasized.

[Supplementary Note 4]

The image processing device according to Supplementary Note 2, wherein the display control means displays, on the display device, the second image and information indicating a relative position of the area based on the dense portion in an entire chest.

[Supplementary Note 5]

The image processing device according to Supplementary Note 1, wherein the deformation means generates, as the information related to the density, distribution information indicating a distribution of the density of the tissue in the area based on the dense portion based on the first image, and deforms the first image based on the distribution information.

[Supplementary Note 6]

The image processing device according to Supplementary Note 5, wherein the deformation means generates information indicating presence or absence of the tissue for each unit area in the area based on the dense portion based on the first image, and generates the distribution information based on the information indicating the presence or absence of the tissue.

[Supplementary Note 7]

The image processing device according to Supplementary Note 5, wherein the deformation means deforms the first image in such a way as to make the density uniform based on the distribution information.

[Supplementary Note 8]

The image processing device according to Supplementary Note 5, wherein the deformation means calculates a deformation vector for each unit area in the area based on the dense portion based on the distribution information, and deforms the first image based on the deformation vector.

[Supplementary Note 9]

The image processing device according to Supplementary Note 1, wherein

the detection means detects the dense portion based on the chest image and an inference model, and
the inference model is a model obtained by performing machine learning of a relationship between the chest image and the dense portion.

[Supplementary Note 10]

An image processing method by a computer, comprising:

detecting a dense portion where tissue is dense from a chest image;
acquiring a first image obtained by cutting out an area based on the dense portion from the chest image;
deforming the first image based on information related to density of the tissue in the first image; and
displaying, on a display device, a second image obtained by deforming the first image.

[Supplementary Note 11]

A storage medium storing a program for causing a computer to execute processing comprising:

detecting a dense portion where tissue is dense from a chest image;
acquiring a first image obtained by cutting out an area based on the dense portion from the chest image;
deforming the first image based on information related to density of the tissue in the first image; and displaying, on a display device, a second image obtained by deforming the first image.

While the invention has been particularly shown and described with reference to example embodiments thereof, the invention is not limited to these example embodiments. It will be understood by those of ordinary skilled in the art that various changes in form and details may be made therein without departing from the spirit and scope of the present invention as defined by the claims. In other words, it is needless to say that the present invention includes various modifications that could be made by a person skilled in the art according to the entire disclosure including the scope of the claims, and the technical philosophy. All Patent and Non-Patent Literatures mentioned in this specification are incorporated by reference in its entirety.

DESCRIPTION OF REFERENCE NUMERALS

| | |
|---|---|
| 1, 1X | Image processing device |
| 2 | Chest image generation device |
| 3 | Display device |
| 4 | Operation device |
| 11 | Processor |
| 12 | Memory |
| 13 | Interface |
| 100 | Chest image processing system |

**Claims**

1. An image processing device comprising:

   a detection means for detecting a dense portion where tissue is dense from a chest image;
   an acquisition means for acquiring a first image obtained by cutting out an area based on the dense portion from the chest image;
   a deformation means for deforming the first image based on information related to density of the tissue in the first image; and
   a display control means for displaying, on a display device, a second image obtained by deforming the first image.

2. The image processing device according to claim 1, wherein the display control means displays, on the display device, the second image and information related to the area based on the dense portion.

3. The image processing device according to claim 2, wherein the display control means displays, on the display device, the second image and the chest image in which the area based on the dense portion is emphasized.

4. The image processing device according to claim 2, wherein the display control means displays, on the display device, the second image and information indicating a relative position of the area based on the dense portion in an entire chest.

5. The image processing device according to claim 1, wherein the deformation means generates, as the information related to the density, distribution information indicating a distribution of the density of the tissue in the area based on the dense portion based on the first image, and deforms the first image based on the distribution information.

6. The image processing device according to claim 5, wherein the deformation means generates information indicating presence or absence of the tissue for each unit area in the area based on the dense portion based on the first image, and generates the distribution information based on the information indicating the presence or absence of the tissue.

7. The image processing device according to claim 5, wherein the deformation means deforms the first image in such a way as to make the density uniform based on the distribution information.

8. The image processing device according to claim 5, wherein the deformation means calculates a deformation vector for each unit area in the area based on the dense portion based on the distribution information, and deforms the first image based on the deformation vector.

9. The image processing device according to claim 1, wherein

   the detection means detects the dense portion based on the chest image and an inference model, and
   the inference model is a model obtained by performing machine learning of a relationship between the chest image and the dense portion.

10. An image processing method by a computer, comprising:

    detecting a dense portion where tissue is dense from a chest image;
    acquiring a first image obtained by cutting out an area based on the dense portion from the chest image;
    deforming the first image based on information related to density of the tissue in the first image; and
    displaying, on a display device, a second image obtained by deforming the first image.

11. A storage medium storing a program for causing a computer to execute processing comprising:

> detecting a dense portion where tissue is dense from a chest image;
> acquiring a first image obtained by cutting out an area based on the dense portion from the chest image;
> deforming the first image based on information related to density of the tissue in the first image; and
> displaying, on a display device, a second image obtained by deforming the first image.

## Fig. 1

100 : CHEST IMAGE PROCESSING SYSTEM

Fig. 2

CHEST IMAGE

DETECT
DENSE PORTION
OF TISSUE

□ :DETECTION AREA OF
DENSE PORTION

CROP DENSE PORTION &
PERFORM DEFORMATION
TO MAKE DENSITY OF
TISSUE UNIFORM

DEFORMED CROP IMAGE

Fig. 3

Fig. 4A

:NON-TARGET TISSUE PIXEL

:TARGET TISSUE PIXEL

Fig. 4B

HIGH                    LOW

TARGET TISSUE DENSITY

Fig. 5A

Fig. 5B

Fig. 6A

| 5 | 8 | 5 | 2 |
|---|---|---|---|
| 5 | 5 | 5 | 2 |
| 2 | 2 | 2 | 2 |
| 0 | 0 | 0 | 0 |

Fig. 6B

Fig. 7

Fig. 8

Fig. 9

UPPER OUTER PART
OF BREAST

UPPER INNER PART
OF BREAST

72

71

74

LOWER OUTER PART
OF BREAST

LOWER INNER PART
OF BREAST

# Fig. 10

```
                    ┌─────────────┐
                    │    START    │
                    └──────┬──────┘
                           │
S11   ┌────────────────────▼────────────────────┐
      │           ACQUIRE CHEST IMAGE           │
      └────────────────────┬────────────────────┘
                           │
S12   ┌────────────────────▼────────────────────┐
      │      DETECT DENSE PORTION OF TISSUE     │
      └────────────────────┬────────────────────┘
                           │
S13          ┌─────────────▼─────────────┐      No
      ◄───────│  IS DENSE PORTION DETECTED? │──────────┐
             └─────────────┬─────────────┘           │
                        Yes │                          │
S14   ┌────────────────────▼────────────────────┐     │
      │           ACQUIRE CROP IMAGE            │     │
      │        BASED ON DETECTION RESULT        │     │
      └────────────────────┬────────────────────┘     │
                           │                          │
S15   ┌────────────────────▼────────────────────┐     │
      │           DEFORM CROP IMAGE             │     │
      │     TO IMPROVE VISIBILITY OF TISSUE     │     │
      └────────────────────┬────────────────────┘     │
                           │                          │
S16   ┌────────────────────▼────────────────────┐  S17 ┌──────────────────────┐
      │              DISPLAY                    │     │  DISPLAY CHEST IMAGE  │
      │      AT LEAST DEFORMED CROP IMAGE       │     │                      │
      └────────────────────┬────────────────────┘     └──────────┬───────────┘
                           │                                     │
                           └─────────────────◄───────────────────┘
                           │
                    ┌──────▼──────┐
                    │     END     │
                    └─────────────┘
```

# Fig. 11

1X

### DETECTION MEANS
31X

### ACQUISITION MEANS
32X

### DEFORMATION MEANS
33X

### DISPLAY CONTROL MEANS
34X

# Fig. 12

```
                          ┌─────────────┐
                          │    START    │
                          └─────────────┘
                                 │
                                 ▼
 S21  ┌──────────────────────────────────────────────────┐
      │   DETECT DENSE PORTION WHERE TISSUE IS DENSE      │
      │              FROM CHEST IMAGE                     │
      └──────────────────────────────────────────────────┘
                                 │
                                 ▼
 S22  ┌──────────────────────────────────────────────────┐
      │   ACQUIRE FIRST IMAGE OBTAINED BY CUTTING OUT     │
      │  AREA BASED ON DENSE PORTION FROM CHEST IMAGE     │
      └──────────────────────────────────────────────────┘
                                 │
                                 ▼
 S23  ┌──────────────────────────────────────────────────┐
      │     DEFORM FIRST IMAGE BASED ON INFORMATION       │
      │  RELATED TO DENSITY OF TISSUE IN FIRST IMAGE      │
      └──────────────────────────────────────────────────┘
                                 │
                                 ▼
 S24  ┌──────────────────────────────────────────────────┐
      │   DISPLAY SECOND IMAGE OBTAINED BY DEFORMING      │
      │                 FIRST IMAGE                       │
      └──────────────────────────────────────────────────┘
                                 │
                                 ▼
                          ┌─────────────┐
                          │     END     │
                          └─────────────┘
```

## INTERNATIONAL SEARCH REPORT

| International application No. |
| --- |
| **PCT/JP2023/015979** |

| A. | CLASSIFICATION OF SUBJECT MATTER |
| --- | --- |

*A61B 6/00*(2006.01)i
FI: A61B6/00 360Z; A61B6/00 350Z

According to International Patent Classification (IPC) or to both national classification and IPC

| B. | FIELDS SEARCHED |
| --- | --- |

Minimum documentation searched (classification system followed by classification symbols)

A61B6/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2021/195084 A1 (HOLOGIC, INC.) 30 September 2021 (2021-09-30) | 1, 5-6, 8-11 |
| | paragraphs [0071]-[0082], fig. 8, etc. | |
| Y | paragraphs [0071]-[0082], fig. 8, etc. | 2-4 |
| Y | JP 2017-80389 A (KONICA MINOLTA, INC.) 18 May 2017 (2017-05-18) | 2-4 |
| | paragraph [0071] | |
| Y | JP 2021-170174 A (EIZO CORP.) 28 October 2021 (2021-10-28) | 3 |
| | paragraphs [0002], [0055], etc. | |
| A | JP 2012-297 A (KONICA MINOLTA MEDICAL & GRAPHIC, INC.) 05 January 2012 (2012-01-05) | 1-11 |
| | entire text, all drawings | |
| A | JP 2021-511186 A (HALL, Per) 06 May 2021 (2021-05-06) | 1-11 |
| | entire text, all drawings | |
| A | JP 2017-192691 A (CANON KABUSHIKI KAISHA) 26 October 2017 (2017-10-26) | 1-11 |
| | entire text, all drawings | |

| ✓ Further documents are listed in the continuation of Box C. | ✓ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **12 June 2023** | **27 June 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
| --- |
| **PCT/JP2023/015979** |

### C. DOCUMENTS CONSIDERED TO BE RELEVANT

| Category* | Citation of document, with indication, where appropriate, of the relevant passages | Relevant to claim No. |
| --- | --- | --- |
| A | US 2008/0159613 A1 (LUO, Hui et al.) 03 July 2008 (2008-07-03)<br>entire text, all drawings | 1-11 |
| A | JP 2019-33966 A (KONICA MINOLTA, INC.) 07 March 2019 (2019-03-07)<br>entire text, all drawings | 1-11 |

Form PCT/ISA/210 (second sheet) (January 2015)

## INTERNATIONAL SEARCH REPORT
### Information on patent family members

International application No.

**PCT/JP2023/015979**

| Patent document cited in search report | | | Publication date (day/month/year) | Patent family member(s) | | | Publication date (day/month/year) |
|---|---|---|---|---|---|---|---|
| WO | 2021/195084 | A1 | 30 September 2021 | JP 2023-519876 A paragraphs [0063]-[0074], fig. 8, etc. KR 10-2022-0144376 A CN 115348837 A | | | |
| JP | 2017-80389 | A | 18 May 2017 | US 2017/0116731 A1 paragraph [0116] | | | |
| JP | 2021-170174 | A | 28 October 2021 | EP 4105882 A1 paragraphs [0002], [0090] WO 2021/210334 A1 CN 115297764 A KR 10-2022-0156050 A | | | |
| JP | 2012-297 | A | 05 January 2012 | (Family: none) | | | |
| JP | 2021-511186 | A | 06 May 2021 | US 2020/0360312 A1 entire text, all drawings WO 2019/145896 A1 CN 111937079 A | | | |
| JP | 2017-192691 | A | 26 October 2017 | US 2017/0301093 A1 entire text, all drawings EP 3236418 A2 CN 107292857 A KR 10-2017-0117324 A | | | |
| US | 2008/0159613 | A1 | 03 July 2008 | WO 2008/088478 A1 | | | |
| JP | 2019-33966 | A | 07 March 2019 | US 2019/0057504 A1 entire text, all drawings CN 109394250 A | | | |

Form PCT/ISA/210 (patent family annex) (January 2015)

**EP 4 699 539 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021170174 A